# EUROPEAN PATENT SPECIFICATION

(11) **EP 1 612 279 B1**
(45) Date of publication and mention of the grant of the patent: **11.03.2009**
(21) Application number: 05013826.2
(22) Date of filing: 27.06.2005
(51) Int. Cl.: C12Q 1/68

(54) **Association of single nucleotide polymorphisms in PPAR gamma with osteoporosis**
Assoziation von SNPs in PPAR gamma mit Osteoporose
Association de SNPs dans PPAR gamma avec la maladie de l'Osteoporose

(30) Priority: 29.06.2004 US 584116 P
(43) Date of publication of application: 04.01.2006
(73) Proprietor: Roche Diagnostics GmbH, 68305 Mannheim (DE); F. Hoffmann-La Roche AG, 4070 Basel (CH)
(72) Inventor: Higuchi, Russell Gene, Alameda, CA 94501 (US); Li, Jia, Union City California 94587 (US); Peltz, Gary Allen, Redwood City California 94062 (US); Ro, Sunshee Kwon, Foster City, CA 94404 (US)

(56) References cited:
- WO-A-01/53311
- DOGAN SONGUEL ET AL: "Association of peroxisome proliferator - activated receptor gamma 2 Pro-12-Ala polymorphism with endometriosis" FERTILITY AND STERILITY, vol. 81, no. 5, May 2004 (2004-05), pages 1411-1413, XP008053748 ISSN: 0015-0282
- YEN CHUNG-JEN ET AL: "Molecular scanning of the human peroxisome proliferator activated receptor gamma (hPPARgamma) gene in diabetic caucasians: Identification of a Pro12Ala PPARgamma2 missense mutation" BIOCHEMICAL AND BIOPHYSICAL RESEARCH COMMUNICATIONS, vol. 241, no. 2, 18 December 1997 (1997-12-18), pages 270-274, XP002348425 ISSN: 0006-291X
- OGAWA S ET AL: "ASSOCIATION OF BONE MINERAL DENSITY WITH A POLYMORPHISM OF THE PEROXISOME PROLIFERATOR-ACTIVATED RECEPTOR GAMMA GENE: PPARGAMMA EXPRESSION IN OSTEOBLASTS" BIOCHEMICAL AND BIOPHYSICAL RESEARCH COMMUNICATIONS, ACADEMIC PRESS INC. ORLANDO, FL, US, vol. 260, no. 1, 1999, pages 122-126, XP000887116 ISSN: 0006-291X
- GRANT S F ET AL: "Reduced bone density and osteoporosis associated with a polymorphic Sp1 binding site in the collagen type I alpha 1 gene." NATURE GENETICS. OCT 1996, vol. 14, no. 2, October 1996 (1996-10), pages 203-205, XP000867512 ISSN: 1061-4036
- MANN VAL ET AL: "A COL1A1 Sp1 binding site polymorphism predisposes to osteoporotic fracture by affecting bone density and quality" JOURNAL OF CLINICAL INVESTIGATION, vol. 107, no. 7, April 2001 (2001-04), pages 899-907, XP002286258 ISSN: 0021-9738
- MIRANDOLA S ET AL: "Rapid and efficient genotype analysis of the COL1A1 Sp1 binding site dimorphism, a genetic marker for bone mineral density" MOLECULAR AND CELLULAR PROBES, ACADEMIC PRESS, LONDON, GB, vol. 16, no. 1, February 2002 (2002-02), pages 73-75, XP004466429 ISSN: 0890-8508
- DATABASE SNP [Online] US NATIONAL LIBRARY OF MEDICINE (NLM), BETHESDA, MD, US; refSNP ID: rs1899951 2 January 2001 (2001-01-02), XP002348470 retrieved from NCBI Database accession no. ss2782890

## Description

### BACKGROUND OF THE INVENTION

Peroxisome proliferator-activated receptor γ (PPARγ) is a member of the nuclear hormone receptor family of transcription factors. PPARγ plays a role in the regulation of adipocyte differentiation and glucose and fatty acid metabolism. It has also been postulated to play a role in bone growth in mouse models (*see, e.g,* Czernik et al., Endocrinology 143:2376-2384, 2002; Akune, et al., J. Clin. Invest 113:846-855, 2004).

A number of single nucleotide polymorphisms (SNPs) have been reported in the PPARγ2 gene. These include the Pro12Ala polymorphism, a C to G nucleotide substitution at nucleotide 34 of the coding region that results in replacement of a proline (Pro) with alanine (Ala) at position 12 of PPARγ2 (Yen et al., Biochem. Biophys. Res. Comm. 241:270-274 (1997). The Ala polymorphism is relatively uncommon. In Caucasians, the ethnic group with the highest frequency, the carrier prevalence is about 25%. The Pro12Ala polymorphism has been associated with diabetes (*e*.*g*., Alshuler et al, Nat. Gen. 26:76-80, 2000; Mori et al., Diabetes 50:891-894, 2001; Douglas et al. Diabetes 50:886-890, 2001) as well as with other diseases, e.g., endometriosis (Dogan et al., Fertility and Sterility 81: 1411-1413, 2004). Initially, it was reported that a 75% risk reduction for diabetes was conferred by the Ala allele (Deeb et al., Nat. Gen. 20:284-287, 1998), but subsequent studies did not reproduce this association. A larger study identified a significant increase in diabetes risk associated with the more common proline allele (85% frequency). Because the risk allele occurs at such high frequency, its modest effect translates into a large population attributable risk-influencing as much as 25% of type 2 diabetes in the general population (Alshuler *et al, supra*).

Another SNP, the VN102 polymorphism, is in strong linkage disequilibrium with the Pro12Ala polymorphism. The frequency of the polymorphic "A" allele for this polymorphism is similar to that of the Ala allele frequency for the Pro12A1a polymorphism. The PPARγ VN102 alleles per se are known (DATABASE SNP, US Nat. Library of Medicine (NLM), ref SNP ID: rs 1899951, 2001-01-02, XP 002348470, Database accession no. ss2782890).

Although another PPARγ2 polymorphism, a C to T transition in exon 6 of *PPARγ2*, has been reported to be associated with bone mineral density in a Japanese population of postmenopausal women (Ogawa et al., Biochm. Biophys. Res. Comm. 260:122-126, 1999), there has been no prior association observed between the Pro12Ala and VN102 polymorphisms and risk for diseases involving bone density, such as osteoporosis.

### BRIEF SUMMARY OF THE INVENTION

The current invention is thus based on the discovery that the PPARγ2 position 12 polymorphism (Pro12Ala) and the VN102 SNP are associated with risk for diseases involving loss of bone density, *e.g*, osteoporosis. Further, the inventors have discovered that these PPARγ polymorphisms interact with the *COL1A* polymorphism in the Sp1 binding site in intron 1 of the collagen type 1 alpha gene. The Sp1-site "T" allele within intron 1 of COL1A1 gene is known to be associated with osteoporosis (Grant et al., Nature Genetics 14: 203-205, 1996; Mann et al., The Journal of Clinical Investigation 107: 899-907,2001; Mivandola et al., Molecular and Cellular Probes 16: 73-75, 2002).

The invention thus provides a method of detecting a propensity of an individual for developing osteoporosis, the method comprising detecting the presence of homozygous PPARγ Pro12A1a alleles or homozygous PPARγVN102 alleles in an individual and recording a diagnosis of an increased risk of osteoporosis. The diagnosis is often recorded on a computer readable form. In one embodiment, the individual is a female. In another embodiment, the individual is a Caucasian.

The Pro12A1a alleles are typically detected by determining the presence of a "C" nucleotide in the first position of codon "CCA" that encodes residue 12 of PPARγ2 in a genomic DNA sample from the individual. The genomic DNA sample is often obtained from blood, but may be obtained from other tissues as well. The method of detecting the polymorphism generally involves an amplification reaction, *e.g*., a polymerase chain reaction (PCR).

In one embodiment, the amplification reaction is performed with a primer set comprising an allele-specific oligonucleotide primer for the Pro allele and an allele-specific oligonucleotide primer for the Ala allele. Exemplary allele-specific oligonucleotides comprise the primer sequences set forth in SEQ ID NOs:1 and 2.

In another embodiment, the amplification reaction comprises a step of hybridizing an amplified product with a labeled probe that specifically binds to the Pro allele or the Ala allele.

In another embodiment, the invention provides a method of determining the presence of a "Pro" allele of the Pro12Ala polymorphism by detecting the presence of a polypeptide comprising Pro at position 12. The allelic variant can be detected, *e.g*., using antibodies such as monoclonal antibodies that specifically binds to the Pro allele.

The presence of a VN102 allele is also detected in a nucleic acid sample that comprises genomic DNA from the individual. In a typical embodiment, the detection step comprises an amplification reaction, often PCR, using a primer set comprising an allele-specific oligonucleotide primer for the "G" allele and an allele-specific oligonucleotide primer for the "A" allele. Exemplary allele-specific oligonucleotides comprise the primer sequences set forth in SEQ ID NOs:4 and 5. In another embodiment, the amplification reaction comprises a step of hybridizing an amplified product with a labeled probe that specifically binds to the G allele or the A allele.

In some embodiments, the method can further comprise a step of performing a bone density test on the individual.

The invention also provides a method of determining the propensity of an individual for developing osteoporosis by a) detecting the presence or absence of homozygous "G" alleles of the Pro12A1a polymorphism and/or the presence or absence of homozygous PPARγVN102 alleles in a subject; and b) detecting the presence or absence of a "T" allele for the *COL1A1* polymorphism, wherein the presence of homozygous PPAR "G" alleles of the Pro 12 Ala polymorphism and or the presence of homozygous VN102 alleles in conjunction with the presence of a *COL1A1* "T" allele increases the risk of osteoporosis. In typical embodiments, the presence or absence of the polymorphic alleles are determined by assessing the genotype using genomic DNA samples. The invention can further comprise a step of recording an increased risk for osteoporosis when homozygous PPAR "G" alleles of the Pro12 Ala polymorphism and/or homozygous VN102 alleles are present in conjunction with a *COL1A1* "T" allele in an individual.

In another aspect, the invention provides a computer readable medium comprising: a) code for obtaining data representing the results of an assay to determine the presence of homozygous Pro alleles of the Pro12Ala polymorphism or homozygous "G" alleles of the VN102 polymorphism in a biological sample; and b) code for determining if the biological sample is associated with an increased risk with osteoporosis using the data representing the results of the assay. In some embodiments, the computer readable medium further comprises code for obtaining data representing the results of an assay to determine the presence of a "T" allele in the *COL1A* polymorphism and code for determined if the sample is at an additional increased risk for osteoporosis using the data representing the results of the assay.

### BRIEF DESCRIPTION OF THE DRAWINGS

Figure 1 shows various known PPARγ SNPs that were screened for an association with osteoporosis.
Figure 2 provides a summary of the genotyping results for the analysis of the Exon 1 C-G polymorphism (Pro12Ala) and its association with conditions, *e.g*., hip fracture; the analysis of the Intron 1, G-A polymorphism (VN102) and its association with conditions, *e*.*g*., hip fracture; and the analysis of the PPARγ and COL1A1 polymorphisms.

### DETAILED DESCRIPTION OF THE INVENTION

### Definitions

The term "allele" refers to a nucleotide sequence variant of a gene of interest.

The terms "Pro12Ala polymorphism" and "Pro12Ala SNP" are used interchangeably to refer to a PPARγ2 polymorphism (CCA/GCA) that occurs in exon 1 in the codon encoding residue 12 of PPARγ2. The standard name is NT_005718.5_52893. This name is based on the contig sequence name and position of the SNP in the contig sequence from the National Center for Biotechnology Information (NCBI). The SNP source is NCBI OMIM 601487.0002; db SNP rs1801282.

As used herein, a "C allele" with respect to a Pro12Ala allelic variant refers to a sequence variant that has a cytosine, "C nucleotide", at nucleotide 34 of the coding region of PPARγ2, which results in the presence of proline at position 12 in the PPARγ2 protein.

A "G allele" with respect to the Pro12Ala allelic variant refers to a sequence variant that contains a guanine, "G" nucleotide", at the polymorphic position, resulting in the presence of alanine at position 12 of *PPARγ*2.

The term "VN102 polymorphism" refers to a SNP that occurs in intron 1 of PPARγ2. The polymorphic position is a "G" residue vs. an "A" residue. The SNP source is db SNP rs1899951.

A "G allele" with respect to the VN102 polymorphism refers to an allelic variant that has a guanine, "G" nucleotide, at the polymorphic position in intron 1 of *PPAR*γ2.

An "A allele" for the VN102 polymorphism refers to the presence of an adenine, "A nucleotide", at the polymorphic position in intron 1 of *PPAR*γ2*.*

A "COL1A1" polymorphism as used herein refers to a polymorphism manifested as a change from a "G" to a "T" in the collagen type 1 alpha 1 (COL1A1) gene. This polymorphism is located in an Sp1 binding site in intron 1 of *COL1A1* and has been associated with reduced bone mineral density (Mann et al., J. Clin. Invest. 107:899-907, 2001; Mann & Ralston, Bone 32:711-717, 2003). The polymorphism alters binding of Sp1 to its recognition sequence. The presence of "T" allele increases the risk for osteoporosis.

The term "genotype" refers to a description of the alleles of a gene contained in an individual or a sample. In the context of this invention, no distinction is made between the genotype of an individual and the genotype of a sample originating from the individual. Although typically a genotype is determined from samples of diploid cells, a genotype can be determined from a sample of haploid cells, such as a sperm cell.

A "polymorphism" refers to the occurrence of two or more genetically determined alternative sequences of a gene in a population. Typically, the first identified allelic form is arbitrarily designated as the reference form and other allelic forms are designated as alternative or variant alleles. The allelic form occurring most frequently in a selected population is sometimes referred to as the wildtype form.

A "single nucleotide polymorphism" or "SNP" is a site of one nucleotide that varies between alleles. Single nucleotide polymorphisms may occur at any region of the gene. In some instance the polymorphism can result in a change in protein sequence, *e*.*g*., the Pro12A1a polymorphism. The change in protein sequence may affect protein function or not.

The term "linkage disequilibrium" as used herein) refers to alleles at different loci that are not associated at random, i.e., not associated in proportion to their frequencies. If the alleles are in positive linkage disequilibrium, then the alleles occur together more often than expected, assuming statistical independence. Conversely, if the alleles are in negative linkage disequilibrium, then the alleles occur together less often than expected assuming statistical independence." "Strong linkage disequilibrium" as used herein refers to a D' value of about 1.

The term "hybridization" refers to the formation of a duplex structure by two single stranded nucleic acids due to complementary base pairing. Hybridization can occur between exactly complementary nucleic acid strands or between nucleic acid strands that contain minor regions of mismatch. As used herein, the term "substantially complementary" refers to sequences that are complementary except for minor regions of mismatch. Typically, the total number of mismatched nucleotides over a hybridizing region is not more than 3 nucleotides for sequences about 15 nucleotides in length. Conditions under which only exactly complementary nucleic acid strands will hybridize are referred to as "stringent" or "sequence-specific" hybridization conditions. Stable duplexes of substantially complementary nucleic acids can be achieved under less stringent hybridization conditions. Those skilled in the art of nucleic acid technology can determine duplex stability empirically considering a number of variables including, for example, the length and base pair concentration of the oligonucleotides, ionic strength, and incidence of mismatched base pairs. Computer software for calculating duplex stability is commercially available from National Biosciences, Inc. (Plymouth, Minn.); the OLIGO version 5 reference manual is incorporated herein by reference.

Stringent, sequence-specific hybridization conditions, under which an oligonucleotide will hybridize only to the exactly complementary target sequence, are well known in the art (*see, e.g.,* the general references provided in the section on detecting polymorphisms in nucleic acid sequences). Stringent conditions are sequence dependent and will be different in different circumstances. Generally, stringent conditions are selected to be about 5°C lower than the thermal melting point (Tm) for the specific sequence at a defined ionic strength and pH. The Tm is the temperature (under defined ionic strength and pH) at which 50% of the base pairs have dissociated. Relaxing the stringency of the hybridizing conditions will allow sequence mismatches to be tolerated; the degree of mismatch tolerated can be controlled by suitable adjustment of the hybridization conditions.

The term "primer" refers to an oligonucleotide that acts as a point of initiation of DNA synthesis under conditions in which synthesis of a primer extension product complementary to a nucleic acid strand is induced, *i*.*e*., in the presence of four different nucleoside triphosphates and an agent for polymerization (i.e., DNA polymerase or reverse transcriptase) in an appropriate buffer and at a suitable temperature. A primer is preferably a single-stranded oligodeoxyribonucleotide. The primer includes a "hybridizing region" exactly or substantially complementary to the target sequence, preferably about 15 to about 35 nucleotides in length. A primer oligonucleotide can either consist entirely of the hybridizing region or can contain additional features which allow for the detection, immobilization, or manipulation of the amplified product, but which do not alter the ability of the primer to serve as a starting reagent for DNA synthesis. For example, a nucleic acid sequence tail can be included at the 5' end of the primer that hybridizes to a capture oligonucleotide.

An "allele-specific" primer, as used herein, is a primer that hybridizes to the target sequence such that the 3' end, usually the 3' nucleotide, of the primer aligns with the polymorphic site of interest and is exactly complementary to one of the alleles at the polymorphic position. As used herein, the primer is "specific for" the allele to which it is exactly complementary at the 3' end. In general, primer extension is inhibited when a mismatch is present at the 3' end of the primer. An allele-specific primer, when hybridized to the exactly complementary allele, is extendable at a greater efficiency.

The same primer, when hybridized to the other allele, is not readily extendable because of the mismatch at the 3' end of the primer in the hybridization duplex. Thus, the use of an allele-specific primer provides allelic discrimination based on whether an appreciable extension product is formed.

The term "probe" refers to an oligonucleotide that selectively hybridizes to a target nucleic acid under suitable conditions.

An "allele-specific" probe contains a "hybridizing region" exactly or substantially complementary to the target sequence, and is exactly complementary to the target sequence at the polymorphic site of interest. A hybridization assay carried out using the probe under sufficiently stringent hybridization conditions enables the selective detection of a specific target sequence. The probe hybridizing region is preferably from about 10 to about 35 nucleotides in length, more preferably from about 15 to about 35 nucleotides in length. The use of modified bases or base analogues which affect the hybridization stability, which are well known in the art, may enable the use of shorter or longer probes with comparable stability. A probe oligonucleotide can either consist entirely of the hybridizing region or can contain additional features which allow for the detection or immobilization of the probe, but which do not significantly alter the hybridization characteristics of the hybridizing region.

The term "target sequence" or "target region" refers to a region of a nucleic acid that is to be analyzed and comprises the polymorphic site of interest.

As used herein, the terms "nucleic acid," "polynucleotide" and "oligonucleotide" refer to primers, probes, and oligomer fragments. The terms are not limited by length and are generic to linear polymers of polydeoxyribonucleotides (containing 2-deoxy-D-ribose), polyribonucleotides (containing D-ribose), and any other N-glycoside of a purine or pyrimidine base, or modified purine or pyrimidine bases. These terms include double- and single-stranded DNA, as well as double- and single-stranded RNA. Oligonucleotides of the invention may be used as primers and/or probes. Thus oligonucleotides referred to herein as "primers" may act as probes and oligonucleotides referred to as "probes" may act as primer in some embodiments.

A nucleic acid, polynucleotide or oligonucleotide can comprise phosphodiester linkages or modified linkages including, but not limited to phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate or sulfone linkages, and combinations of such linkages.

A nucleic acid, polynucleotide or oligonucleotide can comprise the five biologically occurring bases (adenine, guanine, thymine, cytosine and uracil) and/or bases other than the five biologically occurring bases. These bases may serve a number of purposes, e.g., to stabilize or destabilize hybridization; to promote or inhibit probe degradation; or as attachment points for detectable moieties or quencher moieties. For example, a polynucleotide of the invention can contain one or more modified, non-standard, or derivatized base moieties, including, but not limited to, N⁶-methyl-adenine, N⁶-tert-butyl-benzyl-adenine, imidazole, substituted imidazoles, 5-fluorouracil, 5-bromouracil, 5-chlorouracil, 5-iodouracil, hypoxanthine, xanthine, 4-acetylcytosine, 5-(carboxyhydroxymethyl)uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D mannosylqueosine, 5'-methoxycarboxymethyluracil, 5-methoxyuracil, 2-methylthio-N6- isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acidmethylester, 3-(3-amino-3-N-2-carboxypropyl) uracil, (acp3)w, 2,6- diaminopurine, and 5-propynyl pyrimidine. Other examples of modified, non-standard, or derivatized base moieties may be found in U.S. Patent Nos. 6,001,611; 5,955,589; 5,844,106; 5,789,562; 5,750,343; 5,728,525; and 5,679,785, each of which is incorporated herein by reference in its entirety.

Furthermore, a nucleic acid, polynucleotide or oligonucleotide can comprise one or more modified sugar moieties including, but not limited to, arabinose, 2-fluoroarabinose, xylulose, and a hexose.

### Introduction

This invention is based on the discovery of single nucleotide polymorphisms in PPAR-y2 that are associated with an increased risk for disease or condition involving loss of bone density, *e.g*., osteoporosis. A number of genetic variants in PPAR-γ2 have been identified. One of these is a C to G substitution in codon 12 of PPARγ that results in an amino acid change from proline to Ala (CCG (Pro)--> GCG (Ala)). This polymorphism has been associated with Type 2 diabetes and obesity. The less frequent Ala12 variant is estimated to reduce the risk of developing Type 2 diabetes by 20 percent. In the current invention, the presence of homozygous "C" alleles (also referred to herein as the "Pro" allele) is associated with an increased risk for osteoporosis.

Typically, the Pro12Ala polymorphism is detected by determining the presence of the "C" allele or the "G" allele in genomic DNA obtained from a biological sample from an individual. However, the polymorphism may also be detected in other nucleic acid samples, *e.g*., mRNA isolated from a tissue in which PPARγ2 is expressed, *e.g*, adipocytes. Further, the polymorphism can also be detected by analyzing the protein product(s) of the PPARγ2 alleles present in an individual. Exemplary methods of determining the presence of a polymorphic variant are described in further detail below.

Another polymorphism, VN102, is in strong linkage disequilibrium with Pro12Ala. The VN102 polymorphism is a G to A substitution in intron 1. The presence of homozygous "G" allele for VN102 is also associated with an increased risk for diseases or conditions relating to loss of bone density, such as osteoporosis. VN102 polymorphic variants are detected by determining the genotype of an individual at this site.

The invention is also based on the discovery that the PPARγ polymorphisms described herein interact with the *COL1A1* polymorphism in an Sp1 site in intron 1 such that individuals possessing both: 1) a *COL1A1* "T" allele and 2) homozygous PPARγ Pro12 Ala "G" alleles and homozygous VN102 "G" alleles are at an increased risk, *i*.*e*., have a statistically significant greater risk, for osteoporosis relative to having only the *COL1A1* polymorphism or the PPARγ polymorphism(s).

### Detection of nucleic acid sequence polymorphisms

Detection techniques for evaluating nucleic acids for the presence of a SNP involve procedures well known in the field of molecular genetics. Further, many of the methods involve amplification of nucleic acids. Ample guidance for performing is provided in the art. Exemplary references include manuals such as PCR Technology: Principles and Applications for DNA Amplification (ed. H. A. Erlich, Freeman Press, NY, N.Y., 1992); PCR Protocols: A Guide to Methods and Applications (eds. Innis, et al., Academic Press, San Diego, Calif., 1990); Current Protocols in Molecular Biology, Ausubel, 1994-1999, including supplemental updates through April 2004; Sambrook & Russell, Molecular Cloning, A Laboratory Manual (3rd Ed, 2001).

Although the methods typically employ PCR steps, other amplification protocols may also be used. Suitable amplification methods include ligase chain reaction (*see, e.g.,* Wu & Wallace, Genomics 4:560-569, 1988); strand displacement assay (*see, e.g.,* Walker et al., Proc. Natl. Acad. Sci. USA 89:392-396, 1992; U.S. Pat. No. 5,455,166); and several transcription-based amplification systems, including the methods described in U.S. Pat. Nos. 5,437,990; 5,409,818; and 5,399,491; the transcription amplification system (TAS) (Kwoh et al., Proc. Natl. Acad. Sci. USA 86:1173-1177, 1989); and self-sustained sequence replication (3SR) (Guatelli et al., Proc. Natl. Acad. Sci. USA 87:1874-1878, 1990; WO 92/08800). Alternatively, methods that amplify the probe to detectable levels can be used, such as Qβ-replicase amplification (Kramer & Lizardi, Nature 339:401-402, 1989; Lomeli et al., Clin. Chem. 35:1826-1831, 1989). A review of known amplification methods is provided, for example, by Abramson and Myers in Current Opinion in Biotechnology 4:41-47, 1993.

Typically, the detection of the Pro12Ala and/or VN102 genotype of an individual is performed using oligonucleotide primers and/or probes. Similarly, the detection of the *COL1A1* polymorphism is also performed by assessing the genotype of the individual at this site in the *COL1A1* locus. Oligonucleotides can be prepared by any suitable method, usually chemical synthesis. Oligonucleotides can be synthesized using commercially available reagents and instruments. Alternatively, they can be purchased through commercial sources. Methods of synthesizing oligonucleotides are well known in the art (*see, e.g,* Narang et al., Meth. Enzymol. 68:90-99, 1979; Brown et al., Meth. Enzymol. 68:109-151, 1979; Beaucage et al., Tetrahedron Lett. 22:1859-1862, 1981; and the solid support method of U.S. Pat. No. 4,458,066). In addition, modifications to the above-described methods of synthesis may be used to desirably impact enzyme behavior with respect to the synthesized oligonucleotides. For example, incorporation of modified phosphodiester linkages (e.g., phosphorothioate, methylphosphonates, phosphoamidate, or boranophosphate) or linkages other than a phosphorous acid derivative into an oligonucleotide may be used to prevent cleavage at a selected site. In addition, the use of 2'-amino modified sugars tends to favor displacement over digestion of the oligonucleotide when hybridized to a nucleic acid that is also the template for synthesis of a new nucleic acid strand.

The genotype of an individual for the Pro12Ala and/or VN102 polymorphisms can be determined using many detection methods that are well known in the art. Most assays entail one of several general protocols: hybridization using allele-specific oligonucleotides, primer extension, allele-specific ligation, sequencing, or electrophoretic separation techniques, *e.g*., singled-stranded conformational polymorphism (SSCP) and heteroduplex analysis. Exemplary assays include 5' nuclease assays, template-directed dye-terminator incorporation, molecular beacon allele-specific oligonucleotide assays, single-base extension assays, and SNP scoring by real-time pyrophosphate sequences. Analysis of amplified sequences can be performed using various technologies such as microchips, fluorescence polarization assays, and matrix-assisted laser desorption ionization (MALDI) mass spectrometry. Two methods that can also be used are assays based on invasive cleavage with Flap nucleases and methodologies employing padlock probes.

Determination of the presence or absence of a particular PPARγ2 allele is generally performed by analyzing a nucleic acid sample that is obtained from the individual to be analyzed. Often, the nucleic acid sample comprises genomic DNA. The genomic DNA is typically obtained from blood samples, but may also be obtained from other cells or tissues.

It is also possible to analyze RNA samples for the presence of polymorphic alleles. For example, mRNA can be used to determine the genotype of an individual at the Pro12Ala polymorphic site. In this case, the nucleic acid sample is obtained from cells in which the target nucleic acid is expressed, *e.g*., adipocytes. Such an analysis can be performed by first reverse-transcribing the target RNA using, for example, a viral reverse transcriptase, and then amplifying the resulting cDNA; or using a combined high-temperature reverse-transcription-polymerase chain reaction (RT-PCR), as described in U.S. Pat. Nos. 5,310,652; 5,322,770; 5,561,058; 5,641,864; and 5,693,517.

Frequently used methodologies for analysis of nucleic acid samples to detect SNPs are briefly described. However, any method known in the art can be used in the invention to detect the presence of single nucleotide substitutions.

### Allele Specific Hybridization

This technique, also commonly referred to as allele specific oligonucleotide hybridization (ASO) (*e*.*g*., Stoneking et al., Am. J. Hum. Genet. 48:70-382, 1991; Saiki et al., Nature 324, 163-166, 1986; EP 235,726; and WO 89/11548), relies on distinguishing between two DNA molecules differing by one base by hybridizing an oligonucleotide probe that is specific for one of the variants to an amplified product obtained from amplifying the nucleic acid sample. This method typically employs short oligonucleotides, *e.g*., 15-20 bases in length. The probes are designed to differentially hybridize to one variant versus another. Principles and guidance for designing such probe is available in the art, *e.g*., in the references cited herein. Hybridization conditions should be sufficiently stringent that there is a significant difference in hybridization intensity between alleles, and preferably an essentially binary response, whereby a probe hybridizes to only one of the alleles. Some probes are designed to hybridize to a segment of target DNA such that the polymorphic site aligns with a central position (*e*.*g*., in a 15-base oligonucleotide at the 7 position; in a 16-based oligonucleotide at either the 8 or 9 position) of the probe, but this design is not required.

The amount and/or presence of an allele is determined by measuring the amount of allele-specific oligonucleotide that is hybridized to the sample. Typically, the oligonucleotide is labeled with a label such as a fluorescent label. For example, an allele-specific oligonucleotide is applied to immobilized oligonucleotides representing PPARγ2 SNP sequences. After stringent hybridization and washing conditions, fluorescence intensity is measured for each SNP oligonucleotide.

In one embodiment, the nucleotide present at the polymorphic site is identified by hybridization under sequence-specific hybridization conditions with an oligonucleotide probe exactly complementary to one of the polymorphic alleles in a region encompassing the polymorphic site. The probe hybridizing sequence and sequence-specific hybridization conditions are selected such that a single mismatch at the polymorphic site destabilizes the hybridization duplex sufficiently so that it is effectively not formed. Thus, under sequence-specific hybridization conditions, stable duplexes will form only between the probe and the exactly complementary allelic sequence. Thus, oligonucleotides from about 10 to about 35 nucleotides in length, preferably from about 15 to about 35 nucleotides in length, which are exactly complementary to an allele sequence in a region which encompasses the polymorphic site are within the scope of the invention.

In an alternative embodiment, the nucleotide present at the polymorphic site is identified by hybridization under sufficiently stringent hybridization conditions with an oligonucleotide substantially complementary to one of the SNP alleles in a region encompassing the polymorphic site, and exactly complementary to the allele at the polymorphic site. Because mismatches which occur at non-polymorphic sites are mismatches with both allele sequences, the difference in the number of mismatches in a duplex formed with the target allele sequence and in a duplex formed with the corresponding non-target allele sequence is the same as when an oligonucleotide exactly complementary to the target allele sequence is used. In this embodiment, the hybridization conditions are relaxed sufficiently to allow the formation of stable duplexes with the target sequence, while maintaining sufficient stringency to preclude the formation of stable duplexes with non-target sequences. Under such sufficiently stringent hybridization conditions, stable duplexes will form only between the probe and the target allele. Thus, oligonucleotides from about 10 to about 35 nucleotides in length, preferably from about 15 to about 35 nucleotides in length, which are substantially complementary to an allele sequence in a region which encompasses the polymorphic site, and are exactly complementary to the allele sequence at the polymorphic site, are within the scope of the invention.

The use of substantially, rather than exactly, complementary oligonucleotides may be desirable in assay formats in which optimization of hybridization conditions is limited. For example, in a typical multi-target immobilized-probe assay format, probes for each target are immobilized on a single solid support. Hybridizations are carried out simultaneously by contacting the solid support with a solution containing target DNA. As all hybridizations are carried out under identical conditions, the hybridization conditions cannot be separately optimized for each probe. The incorporation of mismatches into a probe can be used to adjust duplex stability when the assay format precludes adjusting the hybridization conditions. The effect of a particular introduced mismatch on duplex stability is well known, and the duplex stability can be routinely both estimated and empirically determined, as described above. Suitable hybridization conditions, which depend on the exact size and sequence of the probe, can be selected empirically using the guidance provided herein and well known in the art. The use of oligonucleotide probes to detect single base pair differences in sequence is described in, for example, Conner et al., 1983, Proc. Natl. Acad. Sci. USA 80:278-282, and U.S. Pat. Nos. 5,468,613 and 5,604,099, each incorporated herein by reference.

The proportional change in stability between a perfectly matched and a single-base mismatched hybridization duplex depends on the length of the hybridized oligonucleotides. Duplexes formed with shorter probe sequences are destabilized proportionally more by the presence of a mismatch. In practice, oligonucleotides between about 15 and about 35 nucleotides in length are preferred for sequence-specific detection. Furthermore, because the ends of a hybridized oligonucleotide undergo continuous random dissociation and re-annealing due to thermal energy, a mismatch at either end destabilizes the hybridization duplex less than a mismatch occurring internally. Preferably, for discrimination of a single base pair change in target sequence, the probe sequence is selected which hybridizes to the target sequence such that the polymorphic site occurs in the interior region of the probe.

The above criteria for selecting a probe sequence that hybridizes to PPARγ2 apply to the hybridizing region of the probe, i.e., that part of the probe which is involved in hybridization with the target sequence. A probe may be bound to an additional nucleic acid sequence, such as a poly-T tail used to immobilize the probe, without significantly altering the hybridization characteristics of the probe. One of skill in the art will recognize that for use in the present methods, a probe bound to an additional nucleic acid sequence which is not complementary to the target sequence and, thus, is not involved in the hybridization, is essentially equivalent to the unbound probe.

Suitable assay formats for detecting hybrids formed between probes and target nucleic acid sequences in a sample are known in the art and include the immobilized target (dot-blot) format and immobilized probe (reverse dot-blot or line-blot) assay formats. Dot blot and reverse dot blot assay formats are described in U.S. Pat. Nos. 5,310,893; 5,451,512; 5,468,613; and 5,604,099; each incorporated herein by reference.

In a dot-blot format, amplified target DNA is immobilized on a solid support, such as a nylon membrane. The membrane-target complex is incubated with labeled probe under suitable hybridization conditions, unhybridized probe is removed by washing under suitably stringent conditions, and the membrane is monitored for the presence of bound probe. A preferred dot-blot detection assay is described in the examples.

In the reverse dot-blot (or line-blot) format, the probes are immobilized on a solid support, such as a nylon membrane or a microtiter plate. The target DNA is labeled, typically during amplification by the incorporation of labeled primers. One or both of the primers can be labeled. The membrane-probe complex is incubated with the labeled amplified target DNA under suitable hybridization conditions, unhybridized target DNA is removed by washing under suitably stringent conditions, and the membrane is monitored for the presence of bound target DNA. A preferred reverse line-blot detection assay is described in the examples.

An allele-specific probe that is specific for one of the polymorphism variants is often used in conjunction with the allele-specific probe for the other polymorphism variant. In some embodiments, the probes are immobilized on a solid support and the target sequence in an individual is analyzed using both probes simultaneously. Examples of nucleic acid arrays are described by WO 95/11995. The same array or a different array can be used for analysis of characterized polymorphisms. WO 95/11995 also describes subarrays that are optimized for detection of variant forms of a pre-characterized polymorphism. Such a subarray can be used in detecting the presence of the Pro12Ala and/or VN102 polymorphisms described herein.

### Allele-Specific Primers

Polymorphisms are also commonly detected using allele-specific amplification or primer extension methods. These reactions typically involve use of primers that are designed to specifically target a polymorphism via a mismatch at the 3' end of a primer.

The presence of a mismatch effects the ability of a polymerase to extend a primer when the polymerase lacks error-correcting activity. For example, to detect an allele sequence using an allele-specific amplification- or extension-based method, a primer complementary to the C allele (or G allele) of the Pro12A1a polymorphism is designed such that the 3' terminal nucleotide hybridizes at the polymorphic position. The presence of the particular allele can be determined by the ability of the primer to initiate extension. If the 3' terminus is mismatched, the extension is impeded. Thus, for example, if a primer matches the "C" allele nucleotide at the 3' end, the primer will be efficiently extended.

Typically, the primer is used in conjunction with a second primer in an amplification reaction. The second primer hybridizes at a site unrelated to the polymorphic position. Amplification proceeds from the two primers leading to a detectable product signifying the particular allelic form is present. Allele-specific amplification- or extension-based methods are described in, for example, WO 93/22456; U.S. Pat. Nos. 5,137,806; 5,595,890; 5,639,611; and U.S. Pat. No. 4,851,331.

Using allele-specific amplification-based genotyping, identification of the alleles requires only detection of the presence or absence of amplified target sequences. Methods for the detection of amplified target sequences are well known in the art. For example, gel electrophoresis and probe hybridization assays described are often used to detect the presence of nucleic acids.

In an alternative probe-less method, the amplified nucleic acid is detected by monitoring the increase in the total amount of double-stranded DNA in the reaction mixture, is described, *e*.*g*., in U.S. Pat. No. 5,994,056; and European Patent Publication Nos. 487,218 and 512,334. The detection of double-stranded target DNA relies on the increased fluorescence various DNA-binding dyes, *e*.*g*., SYBR Green, exhibit when bound to double-stranded DNA.

As appreciated by one in the art, allele-specific amplification methods can be performed in reaction that employ multiple allele-specific primers to target particular alleles. Primers for such multiplex applications are generally labeled with distinguishable labels or are selected such that the amplification products produced from the alleles are distinguishable by size. Thus, for example, both alleles in a single sample can be identified using a single amplification by gel analysis of the amplification product.

As in the case of allele-specific probes, an allele-specific oligonucleotide primer may be exactly complementary to one of the polymorphic alleles in the hybridizing region or may have some mismatches at positions other than the 3' terminus of the oligonucleotide, which mismatches occur at non-polymorphic sites in both allele sequences.

### 5'-nuclease assay

Genotyping can also be performed using a "TaqMan®" or "5'-nuclease assay", as described in U.S. Pat. Nos. 5,210,015; 5,487,972; and 5,804,375; and Holland et al., 1988, Proc. Natl. Acad. Sci. USA 88:7276-7280. In the TaqMan® assay, labeled detection probes that hybridize within the amplified region are added during the amplification reaction. The probes are modified so as to prevent the probes from acting as primers for DNA synthesis. The amplification is performed using a DNA polymerase having 5' to 3' exonuclease activity. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

The hybridization probe can be an allele-specific probe that discriminates between the SNP alleles. Alternatively, the method can be performed using an allele-specific primer and a labeled probe that binds to amplified product.

Any method suitable for detecting degradation product can be used in a 5' nuclease assay. Often, the detection probe is labeled with two fluorescent dyes, one of which is capable of quenching the fluorescence of the other dye. The dyes are attached to the probe, preferably one attached to the 5' terminus and the other is attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Pat. Nos. 5,491,063 and 5,571,673, both incorporated herein by reference, describe alternative methods for detecting the degradation of probe which occurs concomitant with amplification.

### DNA Sequencing and single base extensions

The PPARγ2 SNPs can also be detected by direct sequencing. Methods include *e*.*g*., dideoxy sequencing-based methods and other methods such as Maxam and Gilbert sequence (*see, e.g.,* Sambrook and Russell, *supra*).

Other detection methods include Pyrosequencing^{™} of oligonucleotide-length products. Such methods often employ amplification techniques such as PCR. For example, in pyrosequencing, a sequencing primer is hybridized to a single stranded, PCR-amplified, DNA template; and incubated with the enzymes, DNA polymerase, ATP sulfurylase, luciferase and apyrase, and the substrates, adenosine 5' phosphosulfate (APS) and luciferin. The first of four deoxynucleotide triphosphates (dNTP) is added to the reaction. DNA polymerase catalyzes the incorporation of the deoxynucleotide triphosphate into the DNA strand, if it is complementary to the base in the template strand. Each incorporation event is accompanied by release of pyrophosphate (PPi) in a quantity equimolar to the amount of incorporated nucleotide. ATP sulfurylase quantitatively converts PPi to ATP in the presence of adenosine 5' phosphosulfate. This ATP drives the luciferase-mediated conversion of luciferin to oxyluciferin that generates visible light in amounts that are proportional to the amount of ATP. The light produced in the luciferase-catalyzed reaction is detected by a charge coupled device (CCD) camera and seen as a peak in a pyrogram^{™}. Each light signal is proportional to the number of nucleotides incorporated. Apyrase, a nucleotide degrading enzyme, continuously degrades unincorporated dNTPs and excess ATP. When degradation is complete, another dNTP is added.

Another similar method for characterizing SNPs does not require use of a complete PCR, but typically uses only the extension of a primer by a single, fluorescence-labeled dideoxyribonucleic acid molecule (ddNTP) that is complementary to the nucleotide to be investigated. The nucleotide at the polymorphic site can be identified via detection of a primer that has been extended by one base and is fluorescently labeled (*e*.*g*., Kobayashi et al, Mol. Cell. Probes, 9:175-182, 1995).

### Denaturing Gradient Gel Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution (*see, e*.*g*., Erlich, ed., PCR Technology, Principles and Applications for DNA Amplification, W. H. Freeman and Co, New York, 1992, Chapter 7).

### Single-Strand Conformation Polymorphism Analysis

Alleles of target sequences can be differentiated using single-strand conformation polymorphism analysis, which identifies base differences by alteration in electrophoretic migration of single stranded PCR products, as described, *e.g*, in Orita et al., Proc. Nat. Acad. Sci. 86, 2766-2770 (1989). Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence difference between alleles of target

SNP detection methods often employ labeled oligonucleotides. Oligonucleotides can be labeled by incorporating a label detectable by spectroscopic, photochemical, biochemical, immunochemical, or chemical means. Useful labels include fluorescent dyes, radioactive labels, *e*.*g*., ³²P, electron-dense reagents, enzyme, such as peroxidase or alkaline phsophatase, biotin, or haptens and proteins for which antisera or monoclonal antibodies are available. Labeling techniques are well known in the art (*see, e.g.,* Current Protocols in Molecular Biology, *supra*; Sambrook & Russell, *supra*).

### Detection of protein variants

The Pro12Ala polymorphism results in variant PPARγ2 polypeptide having a Pro at position 12 or an Ala at positions 12. These variant alleles can therefore also be detected by methods that discriminate between the two variant proteins. Often these methods employ an antibody specific to the protein encoded by a variant allele.

A general overview of the applicable technology can be found in Harlow & Lane, Antibodies: A Laboratory Manual (1988) and Harlow & Lane, Using Antibodies (1999). Methods of producing polyclonal and monoclonal antibodies that react specifically with an allelic variant are known to those of skill in the art *(see, e.g.,* Coligan, Current Protocols in Immunology (1991); Harlow & Lane, *supra*; Goding, Monoclonal Antibodies: Principles and Practice (2d ed. 1986); and Kohler & Milstein, Nature 256:495-497 (1975)). Such techniques include antibody preparation by selection of antibodies from libraries of recombinant antibodies in phage or similar vectors, as well as preparation of polyclonal and monoclonal antibodies by immunizing rabbits or mice (*see*, *e.g.,* Huse et al., Science 246:1275-1281 (1989); Ward et al., Nature 341:544-546 (1989)).

The variant alleles can be detected by a variety of immunoassay methods. For a review of immunological and immunoassay procedures, see Basic and Clinical Immunology (Stites & Terr eds., 7th ed. 1991). Moreover, the immunoassays of the present invention can be performed in any of several configurations, which are reviewed extensively in Enzyme Immunoassay (Maggio, ed., 1980); and Harlow & Lane, *supra*. For a review of the general immunoassays, see also Methods in Cell Biology: Antibodies in Cell Biology, volume 37 (Asai, ed. 1993); Basic and Clinical Immunology (Stites & Terr, eds., 7th ed. 1991).

Commonly used assays include noncompetitive assays, *e.g*., sandwich assays, and competitive assays. Typically, an assay such as an ELISA assay can be used. The amount of the polypeptide variant can be determined by performing quantitative analyses.

Other detection techniques, *e.g*., MALDI, may be used to directly detect the presence of a Pro vs. Ala at position 12 of PPARγ2.

### Recording a diagnosis

Either females or males can be analyzed for the presence of the polymorphism. Analysis can be performed at any age. The allelic frequencies may vary in particular populations. Typically, Caucasian populations have the highest Ala allele frequency (about 12%).

As appreciated by one of skill in the art, the methods of the present invention may be used in conjunction with other analyses to detect a propensity for bone density diseases such as osteoporosis. In some embodiments, for example, if a patient exhibits additional risk factors for osteoporosis, the method can comprises an additional step of evaluating bone density.

The methods of the invention typically involve recording the presence of the SNPs associated with a propensity for diseases of bone density. This information may be stored in a computer readable form. Such a computer system typically comprises major subsystems such as a central processor, a system memory (typically RAM), an input/output (I/O) controller, an external device such as a display screen via a display adapter, serial ports, a keyboard, a fixed disk drive via a storage interface and a floppy disk drive operative to receive a floppy disc, and a CD-ROM (or DVD-ROM) device operative to receive a CD-ROM. Many other devices can be connected, such as a network interface connected via a serial port.

The computer system also be linked to a network, comprising a plurality of computing devices linked via a data link, such as an Ethernet cable (coax or 10BaseT), telephone line, ISDN line, wireless network, optical fiber, or other suitable signal transmission medium, whereby at least one network device (e.g., computer, disk array, etc.) comprises a pattern of magnetic domains (e.g., magnetic disk) and/or charge domains (e.g., an array of DRAM cells) composing a bit pattern encoding data acquired from an assay of the invention.

The computer system can comprise code for interpreting the results of a genotype study evaluating the PPARγ2 polymorphic Pro12Ala and VN102 polymorphic alleles. In some embodiments, the computer system also comprises code for interpreting the results of a genotype study evaluating the *COL1A1* intron 1 Sp1 binding site polymorphism. Thus in an exemplary embodiment, the genotype results are provided to a computer where a central processor is executes a computer program for determining the propensity for an increased or decreased risk of conditions involving loss of bone density.

The invention also provides the use of a computer system, such as that described above, which comprises: (1) a computer; (2) a stored bit pattern encoding the genotyping results obtained by the methods of the invention, which may be stored in the computer; (3) and, optionally, (4) a program for determining the risk for disease relating to loss of bone density.

### Methods

One embodiment of the present invention is:

A method of determining a propensity of an individual for developing osteoporosis, the method comprising
a) detecting the presence of homozygous PPARγ Pro12 alleles or homozygous PPARγ VN102 "G" alleles in a sample from the individual; and
b) recording a diagnosis of an increased risk of osteoporosis in case homogeneous PPARγ Pro12 alleles or homogeneous PPARγ VN102 "G" alleles are present.

A further embodiment is a method of detecting a propensity of an individual for developing osteoporosis, the method comprising
a) detecting the presence of homozygous PPARγ Pro 12 alleles or homozygous PPARγ VN102 "G" alleles in the individual; and
b) recording a diagnosis of an increased risk of osteoporosis.

The above methods can further comprise a step of determining whether a *COL1A1* intron 1 Sp1 site "T" allele is present in a sample from the individual.

Preferably, in the above methods the diagnosis is recorded on a computer readable form.

In the above methods the Pro 12 alleles can be detected by determining the presence of a C nucleotide in position 1 of the codon that encodes Pro 12 of PPARγ2 in a genomic DNA sample from the individual, preferably the genomic DNA sample is obtained from blood.

Even more preferably, the step of determining the presence of a C nucleotide comprises an amplification reaction, wherein most preferably the amplification reaction is a polymerase chain reaction.

The amplification reaction can be performed with a primer set comprising an allele-specific oligonucleotide for the Pro allele and an allele-specific oligonucleotide for the Ala allele. The allele-specific oligonucleotide can comprise the primer sequences set forth in SEQ ID NO:1 and 2. In addition, the amplification reaction can comprise a step of hybridizing an amplified product with a labeled probe that specifically binds to the Pro allele or the Ala allele.

Preferably in the above methods, the VN102 alleles can be detected in a genomic DNA from the individual, preferably the genomic DNA sample is obtained from blood.

Even more preferably, the step of determining the presence of the VN102 allele comprises an amplification reaction, wherein most preferably the amplification reaction is a polymerase chain reaction. The amplification reaction can be performed with a primer set comprising an allele-specific oligonucleotide for the "G" allele and an allele-specific oligonucleotide for the "A" allele. The allele-specific oligonucleotide can comprise the primer sequences set forth in SEQ ID NO:4 and 5.

In addition, the amplification reaction can comprise a step of hybridizing an amplified product with a labeled probe that specifically binds to the G allele or the A allele.

Preferably in all of the above methods the individual is female.

Also preferably in all of the above methods the individual is Caucasian.

All these methods can further comprise a step of performing a bone density test on the individual.

### Kits

The invention also provides kits comprising useful components for practicing the methods. In some embodiments, the kit may comprise allele-specific detection probes, which optionally can be fixed to an appropriate support membrane. Such a kit can also contain amplification primers for amplifying a region of the PPARγ2 locus encompassing the polymorphic site. Further, the kit can comprise primers for amplifying a region of the *COL1A1* gene to detect the Sp1 intron 1 polymorphism described herein. Alternatively, useful kits can contain a set of primers comprising an allele-specific primer for the specific amplification of the polymorphic alleles. Such a kit may also comprises probes for the detection of amplification products.

Other optional components of the kits include additional reagents used for genotyping patients. For example, a kit can contain a polymerase, substrate nucleoside triphosphates, means for labeling and/or detecting nucleic acid (for example, an avidin-enzyme conjugate and enzyme substrate and chromogen if the label is biotin), appropriate buffers for amplification or hybridization reactions, and instructions for carrying out the present method.

### EXAMPLES

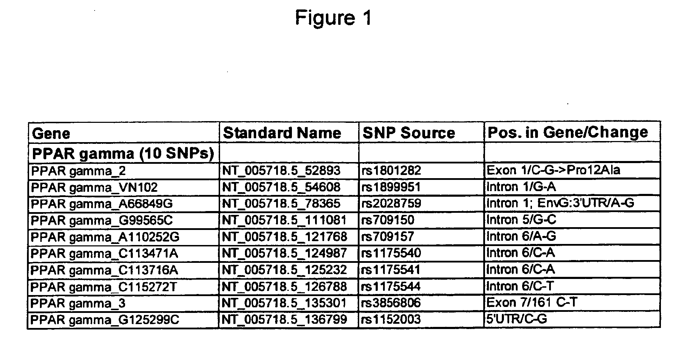

Various known PPARγ SNPs (Figure 1) were screened for an association with osteoporosis. Two SNPs were determined to have an association with risk for hip fracture. Further, the SNPs were determined to have an interaction with the COL1A1 Sp1 polymorphism such that the increased risk was greater when both the PPARγ and COL1A1-osteoporosis-associated genotypes were present. An interactive effect was observed between PPARγ and COL1A1 genotypes on both vertebral fracture and low bone mass density (BMD).

### Example 1. Detection of the Pro12Ala polymorphism association with increased risk of osteoporosis

The association of Pro12Ala polymorphism with risk for osteoporosis was performed by evaluating the presence of the polymorphism in a population of elderly female American patients. Genomic DNA samples from the patients included control samples and samples from elderly women with hip fractures, vertebral fractures, and/or low bone mineral density (BMD). Single nucleotide polymorphisms in PPARγ2 were screened for association with any of these phenotypes.

The analysis for the presence of the "C" and "G" alleles was performed using allele-specific oligonucleotides in a PCR. The allele-specific primer to detect the first allele was 5'-GAAGGAATCGCTTTCTGG-3' (SEQ ID NO:1). The allele-specific primers for the second allele was 5'-GAAGGAATCGCTTTCTGC-3' (SEQ ID NO:2). The query position of the primers is at the 3' end. The common primer used to amplify the target region of *PPARγ2* was 5'-GGTGAAAC TCTGGGAGATTCTCCTA-3' (SEQ ID NO:3). PCR was performed at an annealing temperature of 58°C. Primers were at a concentration of 0.2µM. The reaction was performed in a 50µl volume in a mixture comprising 10 mM Tris HCl pH8.0; 3 mM MgCl₂; 50 µM of each of dATP, dCTP,and dGTP; 25 µM dTTP; 75 uM dUTP, 0.1U UNG, 4% DMSO, 2% glycerol; 0.2X SYBR Green; 6U CEA2 Gold Polymerase; and 3.5-10 ng of human genomic DNA. The reaction was performed using an initial incubation of 50°C 2 min, 95°C 12 min; followed by 45 cycles of 95°C for 20 sec and 58°C for 20 sec.

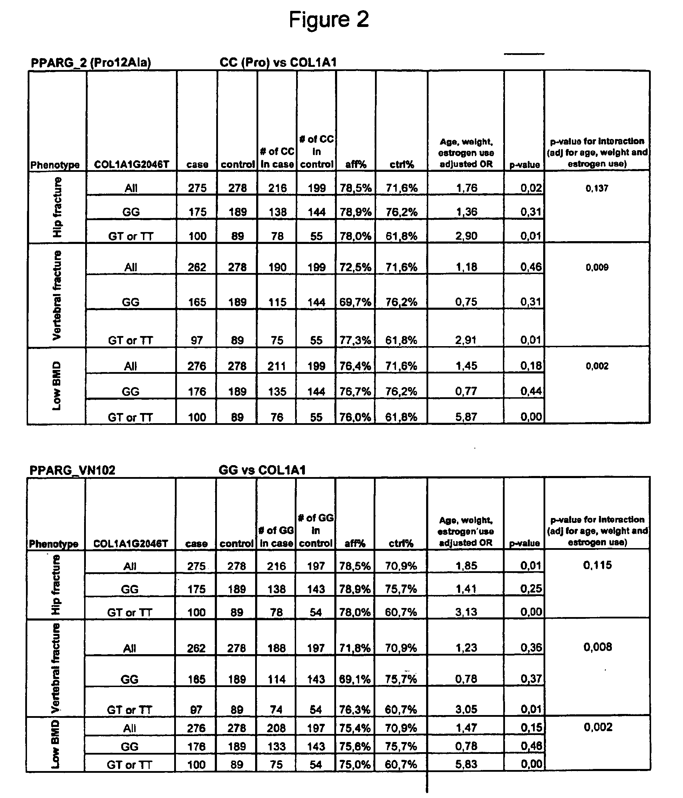

An allelic association with hip fracture was found in the PPARγ gene for the SNP Pro12A1a (also referred to herein as SNP 2) (OMIM 601487.0002) with a p-value of 0.06 and odds ratio of 1.45. After adjustment for age, weight, and estrogen use, the association with Pro12Ala had a p-value of 0.02 and odds ration of 1.76. A summary of the findings is presented in Figure 2.

### Example 2. Detection of the VN102 polymorphism association with increased risk of osteoporosis

The association of the VN102 polymorphism with risk for osteoporosis was performed by evaluating the presence of the polymorphism in the same population of elderly female American patients analyzed above.

The analysis for the presence of the "G" and "A" alleles was performed using allele-specific oligonucleotides in a PCR. The allele-specific primer to detect the first allele was 5'-GTCTTGGGCCTTTAGGAG-3' (SEQ ID NO:4). The allele-specific primers for the second allele was 5'-GTCTTGGGCCTTTAGGAA-3' (SEQ ID NO:5). The query position of the primers is at the 3' end. The common primer used to amplify the target region of *PPARγ2* was 5'-GGCACTG TTTCTCTGTTAAAAATGTG-3' (SEQ ID NO:6). PCR was performed at an annealing temperature of 58°C. Primers were at a concentration of 0.2µM. The reaction was performed in a 50µl volume in a mixture comprising 10 mM Tris HCl pH8.0; 3 mM MgCl₂; 50 µM of each of dATP, dCTP, and dGTP; and 25 µM dTTP; 75 uM dUTP; 0.1U UNG, 4% DMSO, 2% glycerol; 0.2X SYBR Green; 6U CEA2 Gold Polymerase; and 3.5-10 ng of human genomic DNA. The reaction was performed using an initial incubation of 50°C 2 min, 95°C 12 min; followed by 45 cycles of 95°C for 20 sec and 58°C for 20 sec.

An allelic association with hip fracture was found in the PPARγ gene for the SNP VN102 (rs1899951) with a p-value of 0.04 and odds ratio of 1.51. After adjustment for age, weight, and estrogen use, the association with VN102 had a p-value of 0.01 and odds ration of 1.85. A summary of the findings is presented in Figure 2.

### Example 3. Detection of an interactive effect between the Pro12Ala or the VN102 polymorphism with a COL1A1 polymorphism - increased risk of Osteoporosis

A "G" to "T" mutation in an Sp1 binding site in intron 1 of the COL1A1 gene has been reported to be associated with reduced bone mineral density (BMD) in humans. The association of the Pro12Ala or the VN102 polymorphism in combination with this mutation in COL1A1 with risk for osteoporosis was performed by evaluating the presence of these polymorphisms in the same population of elderly female American patients analyzed above.

The analysis for the presence of the "G" and/or "T" alleles was performed using allele-specific oligonucleotides in a PCR. The allele-specific primer to detect the first allele was 5'- CTGCCCAGGGAATGG-3' (SEQ ID NO:7). The allele-specific primer for the second allele was 5'- CCTGCCCAGGGAATGT-3' (SEQ ID NO:8). The query position of the primers is at the 3' end. The common primer used to amplify the target region of *COL1A1* was 5'- AAGGGAGGTCCAGCCCTCAT -3' (SEQ ID NO:9). PCR was performed at an annealing temperature of 58°C. Primers were at a concentration of 0.2µM. The reaction was performed in a 50µl volume in a mixture comprising 10 mM Tris HCl pH8.0; 3 mM MgCl₂; 50 µM of each ofdATP, dCTP, and dGTP; 25 µM dTTP; 75 µM dUTP, 1U UNG, 4% DMSO, 2% glycerol, 0.2X SYBR Green; 6U CEA2 Gold Polymerase; and 3.5-10 ng of human genomic DNA. The reaction was performed using an initial incubation of 50°C 2 min, 95°C 12 min; followed by 45 cycles of 95°C for 20 sec and 58°C for 20 sec.

An interactive effect was observed between *PPAR*γ and *COL1A1* genotypes on both vertebral fracture and low BMD (p=0.009 and 0.002 respectively after adjusting for age, weight, and estrogen use.) There was three fold greater risk (OR=2.9, p=0.01) of vertebral fracture for Pro/Pro genotype among women who had *COL1A1* risk allele after adjusting for age, weight, and estrogen use. A greater risk was also observed for the other two osteoporotic phenotypes. The same effect was also observed for PPARG_VN102. These data indicate a novel interlocus interaction between *PPAR*γ and *COL1A1* and the genetic susceptibility to osteoporosis in older Caucasian women. A summary of the findings is presented in Figure 2.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. A method of determining a propensity of an individual for developing osteoporosis, the method comprising
detecting the presence of homozygous PPARγ Pro12Ala alleles or homozygous PPARγ VN102G alleles in a sample from the individual; and
recording a diagnosis of an increased risk of osteoporosis when homozygous PPARγ Pro12Ala alleles or homozygous PPARγ VN102G alleles are present.

2. The method of claim 1, further comprising a step of determining whether a *COL1A1* intron 1 Sp1 site "T" allele is present in the sample from the individual.

3. The method of claim 1, wherein the diagnosis is recorded on a computer readable form.

4. The method of claim 1, wherein the Pro12Ala alleles are detected by determining the presence of a C nucleotide in position 1 of the codon that encodes Pro12 of PPARγ in a genomic DNA sample from the individual.

5. The method of claim 4, wherein the genomic DNA sample is obtained from blood.

6. The method of claim 4, wherein the step of determining the presence of a C nucleotide comprises an amplification reaction.

7. The method of claim 6, wherein the amplification reaction is a polymerase chain reaction.

8. The method of claim 6, wherein the amplification reaction is performed with a primer set comprising an allele-specific oligonucleotide for the Pro allele and an allele-specific oligonucleotide for the Ala allele.

9. The method of claim 8, wherein the allele-specific oligonucleotide comprise the primer sequences set forth in SEQ ID NO:1 and 2.

10. The method of claim 6, wherein the amplification reaction comprises a step of hybridizing an amplified product with a labeled probe that specifically binds to the Pro allele or the Ala allele.

11. The method of claim 1, wherein the VN102 alleles are detected in a genomic DNA from the individual.

12. The method of claim 11, wherein the genomic DNA sample is obtained from blood.

13. The method of claim 11, wherein the step of determining the presence of the VN102 allele comprises an amplification reaction.

14. The method of claim 13, wherein the amplification reaction is a polymerase chain reaction.

15. The method of claim 13, wherein the amplification reaction is performed with a primer set comprising an allele-specific oligonucleotide for the "G" allele and an allele-specific oligonucleotide for the "A" allele.

16. The method of claim 15, wherein the allele-specific oligonucleotide comprise the primer sequences set forth in SEQ ID NO:4 and 5.

17. The method of claim 6, wherein the amplification reaction comprises a step of hybridizing an amplified product with a labeled probe that specifically binds to the G allele or the A allele.

18. The method of claim 1, wherein the individual is female.

19. The method of claim 1, wherein the individual is Caucasian.

20. The method of claim 1, further comprising a step of performing a bone density test on the individual.

21. A method of determining a propensity of an individual for developing osteoporosis, the method comprising
detecting the presence in a sample of the individual of homozygous PPARγ alleles where the homozygous PPARγ alleles are PPARγ Pro12Ala alleles or PPARγVN102G; and
detecting the presence or absence of a *COL1A1* "T" allele;
wherein the presence of homozygous PPARγ alleles and the presence of a *COL1A1* "T" allele, is indicative of an increased risk of osteoporosis relative to detection of homozygous PPARγ alleles or a *COL1A1* "T" allele alone.

22. A kit for determining a propensity of an individual for developing osteoporosis, the kit comprises
- an amplification primer set comprising an allele-specific oligonucleotide for the PPARγ VN102 "G" allele and an allele-specific oligonucleotide for the PPARγ VN102 "A" allele
- buffers for amplification, wherein the primer set comprises the primer sequences set forth in SEQ ID No. 4 and 5.

## Patentansprüche

1. Verfahren zur Bestimmung einer Neigung eines Individuums zur Entwicklung von Osteoporose, wobei man in dem Verfahren
das Vorliegen homozygoter PPARγ-Pro12Ala-Allele oder homozygoter PPARγ-VN102G-Allele in einer Probe aus dem Individuum nachweist und
eine Diagnose eines erhöhten Osteoporose-Risikos dokumentiert, wenn homozygote PPARγ-Pro12Ala-Allele oder homozygote PPARγ-VN102G-Allele vorliegen.

2. Verfahren nach Anspruch 1, bei dem man in einem weiteren Schritt bestimmt, ob ein *COLIA1*-Intron-1-Sp1-Stelle-"T"-Allel in der Probe aus dem Individuum vorliegt.

3. Verfahren nach Anspruch 1, wobei die Diagnose auf einem computerlesbaren Formblatt dokumentiert wird.

4. Verfahren nach Anspruch 1, wobei die Pro12Ala-Allele nachgewiesen werden, indem man das Vorliegen eines C-Nukleotids in Position 1 des Codons, das Pro12 von PPARγ codiert, in einer genomischen DNA-Probe aus dem Individuum bestimmt.

5. Verfahren nach Anspruch 4, wobei die genomische DNA-Probe aus Blut gewonnen wird.

6. Verfahren nach Anspruch 4, wobei der Schritt der Bestimmung des Vorliegens eines C-Nukleotids eine Amplifikationsreaktion umfasst.

7. Verfahren nach Anspruch 6, wobei es sich bei der Amplifikationsreaktion um eine Polymerasekettenreaktion handelt.

8. Verfahren nach Anspruch 6, wobei die Amplifikationsreaktion mit einem Primersatz ausgeführt wird, der ein allelspezifisches Oligonukleotid für das Pro-Allel und ein allelspezifisches Oligonukleotid für das Ala-Allel umfasst.

9. Verfahren nach Anspruch 8, wobei das allelspezifische Oligonukleotid die Primersequenzen gemäß SEQ ID NO:1 und 2 umfasst.

10. Verfahren nach Anspruch 6, wobei die Amplifikationsreaktion einen Schritt der Hybridisierung eines amplifizierten Produkts mit einer markierten Sonde, die spezifisch an das Pro-Allel oder das Ala-Allel bindet, umfasst.

11. Verfahren nach Anspruch 1, wobei die VN102-Allele in einer genomischen DNA aus dem Individuum nachgewiesen werden.

12. Verfahren nach Anspruch 11, wobei die genomische DNA-Probe aus Blut gewonnen wird.

13. Verfahren nach Anspruch 11, wobei der Schritt der Bestimmung des Vorliegens des VN102-Allels eine Amplifikationsreaktion umfasst.

14. Verfahren nach Anspruch 13, wobei es sich bei der Amplifikationsreaktion um eine Polymerasekettenreaktion handelt.

15. Verfahren nach Anspruch 13, wobei die Amplifikationsreaktion mit einem Primersatz ausgeführt wird, der ein allelspezifisches Oligonukleotid für das "G"-Allel und ein allelspezifisches Oligonukleotid für das "A"-Allel umfasst.

16. Verfahren nach Anspruch 15, wobei das allelspezifische Oligonukleotid die Primersequenzen gemäß SEQ ID NO:4 und 5 umfasst.

17. Verfahren nach Anspruch 6, wobei die Amplifikationsreaktion einen Schritt der Hybridisierung eines amplifizierten Produkts mit einer markierten Sonde, die spezifisch an das G-Allel oder das A-Allel bindet, umfasst.

18. Verfahren nach Anspruch 1, wobei das Individuum weiblich ist.

19. Verfahren nach Anspruch 1, wobei das Individuum kaukasisch ist.

20. Verfahren nach Anspruch 1, bei dem man in einem weiteren Schritt einen Knochendichtetest an dem Individuum vornimmt.

21. Verfahren zur Bestimmung einer Neigung eines Individuums zur Entwicklung von Osteoporose, wobei man in dem Verfahren
das Vorliegen homozygoter PPARγ-Allele in einer Probe des Individuums nachweist, wobei es sich bei den homozygoten PPARγ-Allelen um PPARγ-Pro12Ala-Allele oder PPARγ-VN102G handelt, und
das Vorliegen oder Fehlen eines *COLIA1*-"T"-Allels nachweist,
wobei das Vorliegen homozygoter PPARγ-Allele und das Vorliegen eines *COLIA1*-"T"-Allels ein erhöhtes Osteoporose-Risiko relativ zum jeweils alleinigen Nachweis homozygoter PPARγ-Allele bzw. eines *COLIA1-*"T"-Allels anzeigt.

22. Kit zur Bestimmung einer Neigung eines Individuums zur Entwicklung von Osteoporose, wobei der Kit Folgendes umfasst:
- einen Amplifikationsprimersatz, der ein allelspezifisches Oligonukleotid für das PPARy-VN102-"G"-Allel und ein allelspezifisches Oligonukleotid für das PPARγ-VN102-"A"-Allel umfasst;
- Puffer für die Amplifikation, wobei der Primersatz die Primersequenzen gemäß SEQ ID NO:4 und 5 umfasst.

## Revendications

1. Procédé de détermination d'une propension d'un sujet à développer une ostéoporose, le procédé comprenant
la détection de la présence d'allèles Pro12Ala de PPARγ homozygotes ou d'allèles VN102G de PPARγ homozygotes dans un échantillon provenant du sujet ; et
l'enregistrement d'un diagnostic d'un risque accru d'ostéoporose lorsque des allèles Pro12Ala de PPARγ homozygotes ou des allèles VN102G de PPARγ homozygotes sont présents.

2. Procédé selon la revendication 1, comprenant en outre une étape de détermination si un allèle « T » du site Sp1 de l'intron 1 de *COL1A1* est présent dans l'échantillon provenant du sujet.

3. Procédé selon la revendication 1, dans lequel le diagnostic est enregistré sur un support lisible par un ordinateur.

4. Procédé selon la revendication 1, dans lequel les allèles Pro12Ala sont détectés en déterminant la présence d'un nucléotide C en position 1 du codon qui code pour Pro12 de PPARγ dans un échantillon d'ADN génomique provenant du sujet.

5. Procédé selon la revendication 4, dans lequel l'échantillon d'ADN génomique est obtenu à partir du sang.

6. Procédé selon la revendication 4, dans lequel l'étape de détermination de la présence d'un nucléotide C comprend une réaction d'amplification.

7. Procédé selon la revendication 6, dans lequel la réaction d'amplification est une réaction en chaîne par polymérase.

8. Procédé selon la revendication 6, dans lequel la réaction d'amplification est réalisée avec un jeu d'amorces comprenant un oligonucléotide spécifique d'un allèle pour l'allèle Pro et un oligonucléotide spécifique d'un allèle pour l'allèle Ala.

9. Procédé selon la revendication 8, dans lequel l'oligonucléotide spécifique d'un allèle comprend les séquences d'amorces indiquées dans SEQ ID NO : 1 et 2.

10. Procédé selon la revendication 6, dans lequel la réaction d'amplification comprend une étape d'hybridation d'un produit amplifié avec une sonde marquée qui se lie de manière spécifique à l'allèle Pro ou l'allèle Ala.

11. Procédé selon la revendication 1, dans lequel les allèles VN102 sont détectés dans un ADN génomique provenant du sujet.

12. Procédé selon la revendication 11, dans lequel l'échantillon d'ADN génomique est obtenu à partir du sang.

13. Procédé selon la revendication 11, dans lequel l'étape de détermination de la présence de l'allèle VN102 comprend une réaction d'amplification.

14. Procédé selon la revendication 13, dans lequel la réaction d'amplification est une réaction en chaîne par polymérase.

15. Procédé selon la revendication 13, dans lequel la réaction d'amplification est réalisée avec un jeu d'amorces comprenant un oligonucléotide spécifique d'un allèle pour l'allèle « G » et un oligonucléotide spécifique d'un allèle pour l'allèle « A ».

16. Procédé selon la revendication 15, dans lequel l'oligonucléotide spécifique d'un allèle comprend les séquences d'amorces indiquées dans SEQ ID NO : 4 et 5.

17. Procédé selon la revendication 6, dans lequel la réaction d'amplification comprend une étape d'hybridation d'un produit amplifié avec une sonde marquée qui se lie de manière spécifique à l'allèle G ou l'allèle A.

18. Procédé selon la revendication 1, dans lequel le sujet est féminin.

19. Procédé selon la revendication 1, dans lequel le sujet est Caucasien.

20. Procédé selon la revendication 1, comprenant en outre une étape de réalisation d'un test de densité osseuse sur le sujet.

21. Procédé de détermination d'une propension d'un sujet à développer une ostéoporose, le procédé comprenant
la détection de la présence dans un échantillon du sujet d'allèles de PPARγ homozygotes où les allèles de PPARγ homozygotes sont des allèles Pro12Ala de PPARγ ou VN102G de PPARγ ; et
la détection de la présence ou l'absence d'un allèle « T » de *COL1A1* ;
dans lequel la présence d'allèles de PPARγ homozygotes et la présence d'un allèle « T » de *COL1A1,* est indicatrice d'un risque accru d'ostéoporose par rapport à la détection d'allèles de PPARγ homozygotes ou d'un allèle « T » de *COL1A1* seul.

22. Kit pour déterminer une propension d'un sujet à développer une ostéoporose, le kit comprenant
- un jeu d'amorces pour amplification comprenant un oligonucléotide spécifique d'un allèle pour l'allèle « G » de VN102 de PPARγ et un oligonucléotide spécifique d'un allèle pour l'allèle « A » de VN102 de PPARγ
- des tampons d'amplification, dans lequel le jeu d'amorces comprend les séquences d'amorces indiquées dans SEQ ID No. 4 et 5.


### SEQUENCE LISTING

<110> F. Hoffmann-La Roche AG
   Grenzacherstrasse 124
   4070 Basel
   Switzerland
<120> ASSOCIATION OF SINGLE NUCLEOTIDE
   POLYMORPHISMS IN PPARgamma WITH OSTEOPOROSIS
<130> Case 22482
<150> US 60/584,116
   <151> 2004-06-29
<160> 9
<170> FastSEQ for windows version 4.0
<210> 1
   <211> 18
   <212> DNA
   <213> Artificial Sequence
<220>
   <223> Primer
<400> 1
   gaaggaatcg ctttctgg 18
<210> 2
   <211> 18
   <212> DNA
   <213> Artificial sequence
<220>
   <223> Primer
<400> 2
   gaaggaatcg ctttctgc 18
<210> 3
   <211>
   <212> DNA
   <213> Artificial Sequence
<220>
   <223> Primer
<400> 3
   ggtgaaactc tgggagattc tccta 25
<210> 4
   <211> 18
   <212> DNA
   <213> Artificial sequence
<220>
   <223> Primer
<400> 4
   gtcttgggcc tttaggag 18
<210> 5
   <211> 18
   <212> DNA
   <213> Artificial sequence
<220>
   <223> Primer
<400> 5
   gtcttgggcc tttaggaa 18
<210> 6
   <211> 26
   <212> DNA
   <213> Artificial sequence
<220>
   <223> Primer
<400> 6
   ggcactgttt ctctgttaaa aatgtg 26
<210> 7
   <211> 15
   <212> DNA
   <213> Artificial Sequence
<220>
   <223> Primer
<400> 7
   ctgcccaggg aatgg 15
<210> 8
   <211> 16
   <212> DNA
   <213> Artificial Sequence
<220>
   <223> Primer
<400> 8
   cctgcccagg gaatgt 16
<210> 9
   <211> 20
   <212> DNA
   <213> Artificial sequence
<220>
   <223> Primer
<400> 9
   aagggaggtc cagccctcat 20

### SEQUENCE LISTING

<110> F. Hoffmann-La Roche AG
   Grenzacherstrasse 124
   4070 Basel
   Switzerland
<120> ASSOCIATION OF SINGLE NUCLEOTIDE
   POLYMORPHISMS IN PPARgamma WITH OSTEOPOROSIS
<130> Case 22482
<150> US 60/584,116
   <151> 2004-06-29
<160> 9
<170> FastSEQ for windows version 4.0
<210> 1
   <211> 18
   <212> DNA
   <213> Artificial sequence
<220>
   <223> Primer
<400> 1
   gaaggaatcg ctttctgg 18
<210> 2
   <211> 18
   <212> DNA
   <213> Artificial sequence
<220>
   <223> Primer
<400> 2
   gaaggaatcg ctttctgc 18
<210> 3
   <211> 25
   <212> DNA
   <213> Artificial Sequence
<220>
   <223> Primer
<400> 3
   ggtgaaactc tgggagattc tccta 25
<210> 4
   <211> 18
   <212> DNA
   <213> Artificial Sequence
<220>
   <223> Primer
<400> 4
   gtcttgggcc tttaggag 18
<210> 5
   <211> 18
   <212> DNA
   <213> Artificial sequence
<220>
   <223> Primer
<400> 5
   gtcttgggcc tttaggaa 18
<210> 6
   <211> 26
   <212> DNA
   <213> Artificial Sequence
<220>
   <223> Primer
<400> 6
   ggcactgttt ctctgttaaa aatgtg 26
<210> 7
   <211> 15
   <212> DNA
   <213> Artificial Sequence
<220>
   <223> Primer
<400> 7
   ctgcccaggg aatgg 15
<210> 8
   <211> 16
   <212> DNA
   <213> Artificial sequence
<220>
   <223> Primer
<400> 8
   cctgcccagg gaatgt 16
<210> 9
   <211> 20
   <212> DNA
   <213> Artificial sequence
<220>
   <223> Primer
<400> 9
   aagggaggtc cagccctcat 20